# EUROPEAN PATENT APPLICATION

(11) **EP 2 484 296 A1**
(43) Date of publication of application: **08.08.2012**
(21) Application number: 10818804.6
(22) Date of filing: 22.09.2010
(51) Int. Cl.: A61B 17/16, A61B 17/56, B23B 39/14

(54) **REMOTE OPERATION ACTUATOR AND PLASTIC FORMING METHOD FOR ATTITUDE OPERATION MEMBER**

(30) Priority: 28.09.2009 JP 2009222304
(71) Applicant: NTN Corporation, Osaka-shi, Osaka 550-0003 (JP)
(72) Inventor: NISHIO, Yukihiro, Iwata-shi Shizuoka 438-0037 (JP); NAGANO, Yoshitaka, Iwata-shi Shizuoka 4380037 (JP)
(74) Representative: Hirsch & Associés
(86) International application number: PCT/JP2010/066387
(87) International publication number: WO 2011/037131

(57) **Abstract**

A remote controlled actuator includes an elongated guide section (3) of a curved shape, a distal end member (2) fitted to a tip of the guide section for alteration in attitude, and a work tool (1) provided in the distal end member. A drive shaft (22) for transmitting a driving force of a work tool drive source (41) to the work tool and a curved guide hole (30a) extending to its opposite ends are accommodated inside the guide section. A wire-like attitude altering member (31) is inserted within the guide hole for selective advance or retraction and then is driven by an attitude altering drive source (42) to alter the attitude of the distal end member. The attitude altering member has a curved shape identical with the curved shape of the guide hole under a natural condition while it has not yet been inserted into the guide hole.

## Description

### CROSS REFERENCE TO THE RELATED APPLICATION

This application is based on and claims Convention priority to Japanese patent application No. 2009-222304, filed September 28, 2009, the entire disclosure of which is herein incorporated by reference as a part of this application.

### BACKGROUND OF THE INVENTION

### (Field of the Invention)

The present invention relates to a remote controlled actuator capable of altering the attitude of a work tool, provided at a tip of an elongated guide section of a curved configuration, by remote control and also to a plastic forming method for making an attitude altering member which is one of component parts of such remote controlled actuator.

### (Description of Related Art)

A remote controlled actuator of a type for use in a cutting process in the medical application or in the mechanical application has long been known. This remote controlled actuator is of a structure in which a work tool such as, for example, a tool or a holder element is provided at a tip of an elongated guide section of a rectilinear configuration or a curved configuration and this work tool is controlled by remote control. Hereinafter, in discussing over the problems inherent in the conventional art of the remote controlled actuator, reference will be made to the use of the conventional remote controlled actuator in the medical field in processing a bone.

In the orthopedic field, the artificial joint replacement is well known, in which a joint, of which bone has been abraded by due to bone deterioration, is replaced with an artificial joint. The joint replacement surgery requires a living bone of a patient to be processed to enable an artificial joint to be implanted. In order to enhance the strength of postoperative adhesion between the living bone and the artificial joint, such processing is required to be performed precisely and accurately in conformity to the shape of the artificial joint.

By way of example, during the hip joint replacement surgery, a thigh bone is opened to secure access of an artificial joint into the femoral marrow cavity. In order to secure a strength of contact between the artificial joint and the bone, surfaces of contact of the artificial joint and the bore must be large and so the opening for insertion of the artificial joint is processed to represent an elongated shape extending deep into the bone. In this procedure, a remote controlled actuator is used of a type having such a structure that a tool is rotatably provided in a tip of an elongated guide section so that the tool can be rotated by remote control. The surgical operation for artificial joint replacement generally accompanies skin incision and muscular scission. In other words, the human body must be invaded. In order to minimize the postoperative trace, it is quite often desirable that the elongated guide referred to above is not necessarily straight, but is moderately curved.

To meet this desire, the following technique has hitherto been suggested. For example, the Patent Document 1 listed below discloses the elongated pipe having its intermediate portion curved twice to displace an axial position of the distal end of the pipe relative to the longitudinal axis of the proximal end of the same pipe. To make the axial position of the distal end of the pipe relative to the longitudinal axis of the proximal end of the same pipe is also known from other publications. Also, the Patent Document 2 listed below discloses the elongated pipe rotated by 180°.

### [Prior Art Literature]

[Patent Document 1] United States Patent No. 4,466,429
[Patent Document 2] United States Patent No. 4,265,231

Since the above described remote controlled actuator conventionally employed in the medical field merely controls the rotation of the tool by remote control, accurate positioning of the tool at a site deep into the hole for insertion of the artificial joint and processing of the bone to a complicated shape have been difficult to achieve. In view of this, in order to alleviate the foregoing problems and inconveniences, the attempt has been made, in which the distal end member for rotatably supporting the tool is provided at the tip of the guide section for alteration in the attitude and the attitude of the distal end member is made alterable by remote control. More specifically, the attempt has been made in which a guide hole having its opposite end opening is provided within the guide section, an attitude altering member of a wire-like shape is inserted within this guide hole for selective advance or retraction and a working force for the attitude alteration is applied to the distal end member through this attitude altering member so that the distal end member can be altered in attitude. Since the attitude altering member of the wire-like shape is flexible, the working force necessary to alter the attitude of the distal end member can be transmitted to the distal end member even in the case of the guide pipe and the guide hole being curved.

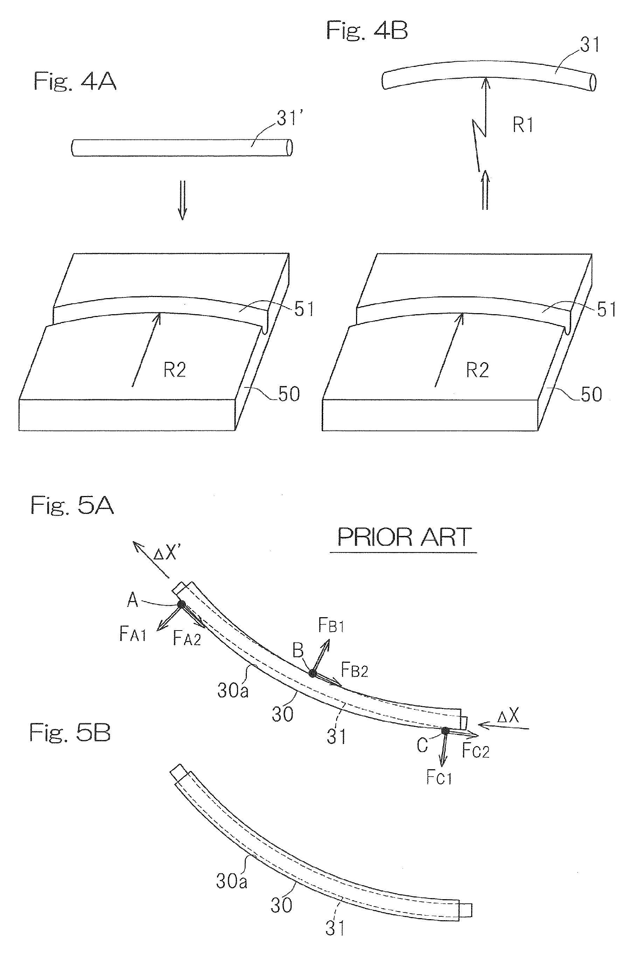

It has, however, been found that the above described construction involves the following problems particularly where the guide hole is of a curved shape. In other words, as shown in Fig. 5A, if the attitude altering member 31 of the wire-like shape, which is straight in a natural condition, is passed within the guide hole 30a of a curved shape, the attitude altering member 31 exerts a resilient restoring force tending to restore it back to the original straight condition and, therefore, contact sites A, B and C are created where a surface of the attitude altering member 31 and an inner wall surface of the guide hole 30a strongly contact each other. Reference characters F_{A1}, F_{B1} and F_{C1}, shown in Fig. 5A represent respective urging forces with which the attitude altering member 31 presses the inner wall surface of the guide hole 30a by the effect of the resilient restoring force. During the selective advance or retraction of the attitude altering member 31, due to the contact forces F_{A1}, F_{B1} and F_{C1} referred to above, frictional forces F_{A2}, F_{B2} and F_{C2} are developed between the attitude altering member 31 and the inner wall surface of the guide hole 30a. Those frictional forces F_{A2}, F_{B2} and F_{C2} tend to increase with an increase of the curvature of the curved shape of the guide hole 30a.

If the attitude altering member 31 is selectively advanced or retracted by an attitude altering drive source, the frictional forces F_{A2}, F_{B2} and F_{C2} referred to above act as respective resisting forces to the attitude altering member 31, causing the attitude altering member 31 to expand and contract. In other words, a displacement ΔX on an input side of the attitude altering member 31 is different from a displacement ΔX' on an output side thereof. In view of the structure of the remote controlled actuator, it is difficult to directly detect the attitude of the distal end member, and, therefore, to control the attitude of the distal end member from the amount of activation of the attitude altering drive source may be considered to be a practically realizable control method. However, if such control method is to be employed, expansion and contraction of the attitude altering member 31, which take place by the reason as hereinabove described, will results in an error represented by the increment and decrement (ΔX - ΔX') and, therefore, a control to alter the attitude of the distal end member highly accurately cannot be accomplished. Also, in order to selectively advance or retract the attitude altering member 31 while overcoming the frictional forces F_{A2}, F_{B2} and F_{C2}, the attitude altering drive source is required to have a large driving force and this leads to an increase of the size of the attitude altering drive source.

### SUMMARY OF THE INVENTION

Therefore, a first object of the present invention herein disclosed is to provide a remote controlled actuator capable of accurately altering the attitude of a work tool, provided at the tip of the elongated guide section of a curved shape, by remote control, which actuator requires a compact drive source for altering the attitude of the work tool. A second object of the present invention herein disclosed is to provide a plastic forming method capable of easily shaping the attitude altering member, which is one of component parts of the remote controlled actuator, to a proper shape that suits to the shape of the guide section.

The remote controlled actuator designed in accordance with the present invention includes an elongated guide section of a curved shape, a distal end member fitted to a tip of the guide section for alteration in attitude, a work tool provided in the distal end member, a work tool drive source for driving the work tool and an attitude altering drive source for operating the attitude of the distal end member. In such case, the guide section has an interior, in which a drive shaft for transmitting a driving force of the work tool drive source to the work tool and a curved guide hole extending to its opposite ends are accommodated, and a wire-like attitude altering member is inserted within the guide hole for selective advance or retraction, with the wire-like attitude altering member being adapted to be driven by the attitude altering drive source to undergo a selective advance or retraction to alter the attitude of the distal end member. In such case, the attitude altering member has a curved shape similar to the curved shape of the guide hole under a natural condition while the attitude altering member has not yet been inserted into the guide hole.

According to the above described construction, as a result of rotation of the work tool fitted to the distal end member, a predetermined work takes place. In such case, when the attitude altering member is selectively advanced and retracted one at a time by the attitude altering drive source, the tip end of the attitude altering member works on the distal end member to allow the attitude of the distal end member, fitted to the tip end of the guide section through the distal end member connecting unit for alteration in attitude, to alter. The attitude altering drive source is provided at a position distant from the distal end member and the alteration of the attitude of the distal end member is carried out by remote control. Since the attitude altering member is passed through the guide hole, the attitude altering member can work on the distal end member properly at all time without being displaced in a direction transverse to the longitudinal direction thereof, and the operation to alter the attitude of the distal end member takes place accurately. Also, since the attitude altering member is in the form of a wire and flexible, the attitude altering operation takes place assuredly even when the guide section is of a type having a curved portion.

Since the attitude altering member is of a curved shape similar to the curved shape of the guide hole, when the attitude altering member has not yet been inserted into the guide hole and is under the natural condition, the frictional force developed between the attitude altering member and the inner wall surface of the guide hole, when in a condition with the attitude altering member having been inserted into the guide hole, is so low that extension or contraction of the attitude altering member will hardly occur. For this reason, an error due to such an extension or a contraction of the attitude altering member becomes small, and therefore, the attitude of the distal end member can accurately be altered. Also, the frictional force between the attitude altering member and the inner wall surface of the guide hole is so low that no large force may be required to selectively advance or retract the attitude altering member and, hence, the use of the attitude altering drive source that is small in size is sufficient.

In the present invention, the attitude altering drive source may be operable to selectively advance or retract the attitude altering member so that the tip of the attitude altering member may press the distal end member, and the use may be made of a counterforce applying unit for applying a force to the distal end member, which force counteracts against a force applied to the distal end member as a result of the selective advance or retraction of the attitude altering member, so that the attitude of the distal end member can be altered and maintained by the balance between the force, which the attitude altering member applies to the distal end member, and the force which the counterforce applying unit applies to the distal end member.

Alternatively, the attitude altering drive source may be operable to selectively advance or retract the attitude altering member so that the tip of the attitude altering member may pull the distal end member, and the use may be made of a counterforce applying unit for applying a force to the distal end member, which force counteracts against a force applied to the distal end member as a result of the selective advance or retraction of the attitude altering member, so that the attitude of the distal end member can be altered and maintained by the balance between the force, which the attitude altering member applies to the distal end member, and the force which the counterforce applying unit applies to the distal end member. According to either of them, the attitude of the distal end member can be accurately altered by means of the attitude altering member.

In the present invention, the attitude altering member may be shaped to the same shape as the curved shape of the guide hole by means of either a plastic deformation caused by a plastic forming or a cutting process.

In the present invention, where the drive shaft is a rotary shaft, a plurality of rolling bearings may be provided for rotatably supporting the drive shaft within the guide section, along with the use of a spring element provided between the neighboring rolling bearings for applying a preload to those rolling bearings. By way of example, where the work tool is a rotary tool and an article to be processed is to be cut by rotating this tool, it is recommended to rotate the tool at a high speed in order to give rise to an authentic finish of the processing. Rotating the tool at the high speed also brings about such an effect as to reduce the cutting resistance acting on the tool. The high speed rotation of the tool effects a reduction in cutting resistance of the tool. Since the rotational force is transmitted to the tool through the thin drive shaft within the guide section, it is necessary to apply the preload to the rolling bearings used to support the drive shaft in order to realize the high speed rotation of the tool. Therefore, if the spring element for the preloading is provided between the neighboring rolling bearings, the spring element can be provided without increasing the diameter of the guide section.

In the present invention, the distal end member may be connected with the tip of the guide section through a distal end member connecting unit for tilting in an arbitrary direction, in which case each of the guide hole and the attitude altering member, inserted into the guide hole, is provided at three or more locations about a center of tilting of the distal end member. In such case, the attitude altering drive source is provided for each of the attitude altering members and the attitude of the distal end member is altered and maintained in two axis directions by the balance between respective forces which the attitude altering members at those three or more locations apply to the distal end member. If the attitude of the distal end member can be altered and maintained in the two axis directions, the work tool provided in the distal end member can be accurately positioned. Also, since the force for altering and maintaining the attitude is applied from the three or more attitude altering members to the distal end member, the attitude stability of the distal end member can be increased.

In the present invention, one or both of the work tool drive source and the attitude altering drive source may be provided within a drive unit housing to which a base end of the guide section is connected. If one or both of the work tool drive source and the attitude altering drive source is/are provided within the drive unit housing, the number of component parts provided outside the drive unit housing can be reduced to thereby simplify the structure of the remote controlled actuator in its entirety.

Also, the work tool drive source and the attitude altering drive source may be provided outside the drive unit housing to which a base end of the guide section is connected. If the work tool drive source and the attitude altering drive source are provided outside the drive unit housing, the drive unit housing can be compactized. For this reason, the handlability at the time the remote controlled actuator is operated with the drive unit housing carried by an operator can be increased.

A plastic forming method for making the attitude altering member for use in the remote controlled actuator designed in accordance with the present invention makes use of a mold having a groove of a sectional shape with a curvature greater than the curvature of the curved shape of the guide hole and capable of accommodating the attitude altering member in a non-movable fashion, with the attitude altering member, which is straight under a natural condition, being plastically deformed while accommodated within the groove in the mold. By inserting the attitude altering member of a straight shape under the natural condition within the groove in the mold, the attitude altering member undergoes an elastic deformation and is then shaped to the target curved shape. The reason for choosing the curvature of the groove that is greater than the curvature of the curved shape of the guide hole is that since the attitude altering member, when removed out of the groove, will restore towards the original shape by a quantity it has been elastically deformed, the attitude altering member need be curved too much by that quantity the attitude altering member has been elastically deformed. It is to be noted that the curvature of the curved shape should be broadly construed as including the extent to which the entirety is curved, not limited as meaning the curvature of the whole or various portions. Accordingly, the shape of the guide hole may not be necessarily limited strictly to an arcuate shape, but may be a shape represented by a quadratic curve or a curved shape similar to the quadratic curve.

### BRIEF DESCRIPTION OF THE DRAWINGS

In any event, the present invention will become more clearly understood from the following description of preferred embodiments thereof, when taken in conjunction with the accompanying drawings. However, the embodiments and the drawings are given only for the purpose of illustration and explanation, and are not to be taken as limiting the scope of the present invention in any way whatsoever, which scope is to be determined by the appended claims.
In the accompanying drawings, like reference numerals are used to denote like parts throughout the several views, and:
Fig. 1 is a diagram showing a schematic structure of a remote controlled actuator designed in accordance with a first preferred embodiment of the present invention;
Fig. 2A is a longitudinal sectional view showing a distal end member and a guide section both employed in the remote controlled actuator;
Fig. 2B is a cross sectional view taken along the line IIB-IIB in Fig. 2A;
Fig. 2C is a diagram showing a connecting structure between the distal end member and a drive shaft;
Fig. 2D is a schematic transverse sectional view showing a rotation preventing mechanism;
Fig. 3A is a diagram showing a front elevational view of a work tool drive mechanism and an attitude altering drive mechanism, both employed in the remote controlled actuator, shown together with a control system;
Fig. 3B is a cross sectional view taken along the line IIIB-IIIB in Fig. 3A;
Fig. 4A is a perspective view showing a mold for plastically forming the attitude altering member of the remote controlled actuator and the attitude altering member before the plastic forming;
Fig. 4B is a perspective view showing the attitude altering member after the plastic forming;
Fig. 5A is an explanatory diagram showing the relation between the conventional guide pipe and the attitude altering member;
Fig. 5B is an explanatory diagram showing the relation between a guide pipe, employed in the practice of the present invention, and the attitude altering member;
Fig. 6A is a longitudinal sectional view showing the distal end member and the guide section both employed in the remote controlled actuator designed in accordance with a second preferred embodiment of the present invention;
Fig. 6B is a cross sectional view taken along the line VIB-VIB in Fig. 6A;
Fig. 6C is a schematic transverse sectional view showing the rotation preventing mechanism;
Fig. 7A is a longitudinal sectional view showing the distal end member and the guide section both employed in the remote controlled actuator designed in accordance with a third preferred embodiment of the present invention;
Fig. 7B is a cross sectional view taken along the line VIIB-VIIB in Fig. 7A;
Fig. 7C is a schematic transverse sectional view showing the rotation preventing mechanism;
Fig. 8A is a longitudinal sectional view showing the distal end member and the guide section both employed in the remote controlled actuator designed in accordance with a forth preferred embodiment of the present invention;
Fig. 8B is a cross sectional view taken along the line VIIIB-VIIIB in Fig. 8A;
Fig. 8C is a schematic transverse sectional view showing the rotation preventing mechanism;
Fig. 9 is a side view showing the work tool drive mechanism and the attitude altering drive mechanism both employed in the remote controlled actuator;
Fig. 10 is a diagram showing a schematic structure of the remote controlled actuator designed in accordance with a fifth preferred embodiment of the present invention;
Fig. 11 is a side view, with a portion cut out, showing respective structures of the work tool drive mechanism and the attitude altering drive mechanism both employed in the remote controlled actuator;
Fig. 12 is a sectional view showing a work tool cable employed in the work tool drive mechanism; and
Fig. 13 is a sectional view showing an attitude altering cable employed in the attitude altering drive mechanism.

### DESCRIPTION OF PREFERRED EMBODIMENTS

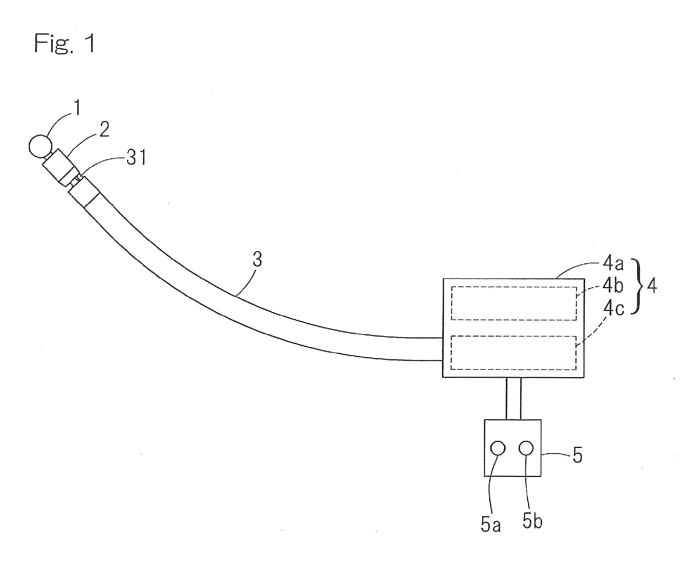

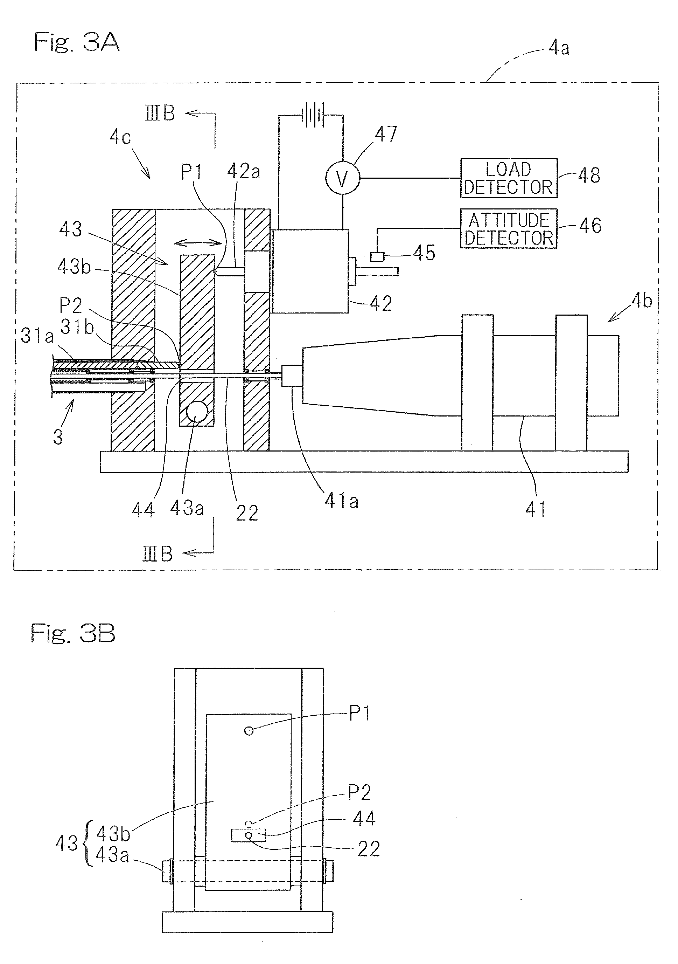

A first preferred embodiment of the present invention will first be described in detail with particular reference to Fig. 1 to Figs. 3A and 3B. Referring to Fig. 1, a remote controlled actuator includes a distal end member 2 for holding a rotary tool 1 or a work tool, a guide section 3 of an elongated shape having a tip to which the distal end member 2 is fitted for alteration in attitude, a drive unit housing 4a to which a base end of the guide section 3 is connected, and a controller 5 for controlling a work tool drive mechanism 4b and an attitude altering drive mechanism 4c, both accommodated within the drive unit housing 4a. In the instance as shown, the guide section 3 has its contour representing a shape similar to a round rod and is of a curved shape, in which respective curvatures of various positions thereof are of a substantially uniform arcuate shape. The drive unit housing 4a forms a drive unit 4 together with the built-in work tool drive mechanism 4b and the similarly built-in attitude altering drive mechanism 4c.

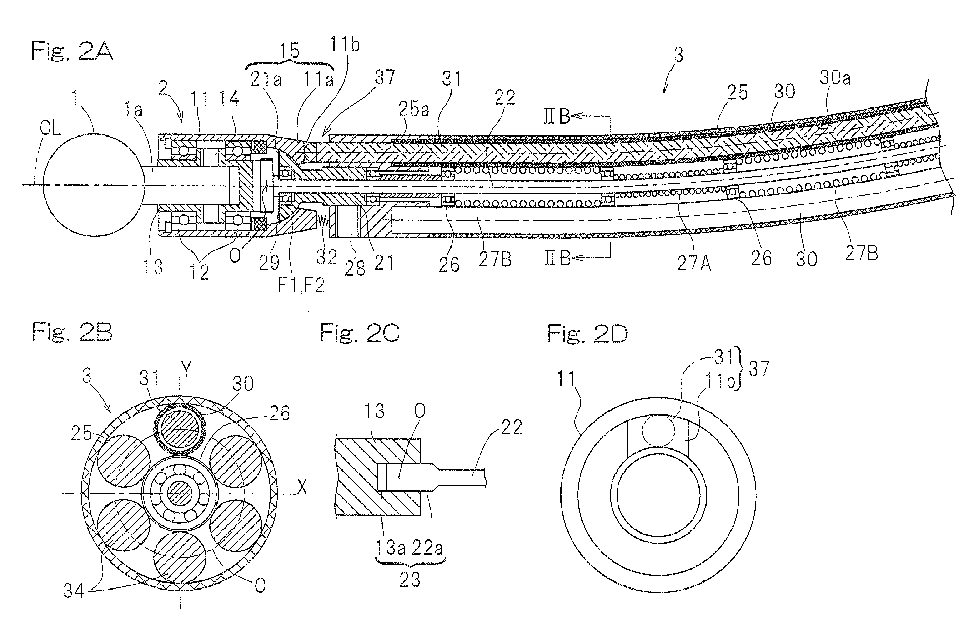

As shown in Fig. 2A, The distal end member 2 includes a generally or substantially cylindrical housing 11 and a spindle 13 rotatably accommodated within such cylindrical housing 11 through a pair of bearings 12. The spindle 13 is of a tubular shape having a distal side opening and has a hollow defined therein, and a tool 1 is drivingly coupled with the spindle 13. Specifically, a shank portion 1a of the tool 1 is inserted into the hollow of the spindle 13 and is then coupled with such spindle 13 by means of a stop pin 14 for rotation together with the spindle 13. The distal end member 2 of the structure described above is coupled with a distal end of the guide section 3 through a distal end member coupling unit 15. The distal end member coupling unit 15 supports the distal end member 2 for displacement in attitude and is comprised of a spherical bearing. More specifically, the distal end member coupling unit 15 includes a guided member 11a in the form of an inner diameter reduced portion at a base end of the housing 11, and a guide member 21a in the form of a collar integral with a constraint member 21 fixed to the tip of the guide section 3. The guided member 11a and the guide member 21a have respective guide faces F1 and F2 that are held in sliding contact with each other, and those guide faces F1 and F2 have respective centers of curvature lying at a point O on the center line or longitudinal axis CL of the spindle 13, having their diameters being reduced towards the base end of the spindle 13. Accordingly, not only can the distal end member 2 be immovably constrained relative to the guide section 3, but it can also be supported for displacement in attitude so that the attitude of the distal end member 2 can be altered. In the instance as shown, since the construction is so employed that the distal end member 2 can alter its attitude about an X-axis passing through the center O of curvature, the guide faces F1 and F2 may be cylindrical surface each having its longitudinal axis represented by the X-axis passing through the center O of curvature.

The guide section 3 includes a drive shaft 22 for transmitting a rotational force exerted by a work tool drive source 41 (Fig. 3A) accommodated within the drive unit housing 4a. In the illustrated example, the drive shaft 22 is employed in the form of a wire capable of undergoing deformation to a certain extent. Material for the wire includes, for example, metal, resin or glass fiber. The wire may be either a single wire or a twisted wire. As best shown in Fig. 2C, the spindle 13 and the drive shaft 22 are coupled together by means of a universal joint 23 for transmitting rotation from the drive shaft 22 to the spindle 13. The universal joint 23 is made up of a groove 13a, defined in a closed base end of the spindle 13, and of a projection 22a defined in a distal end of the drive shaft 22 and engageable in the groove 13a. The center of joint between the groove 13a and the projection 22a is located at the same position as the centers of curvature O of the guide faces F1 and F2. It is to be noted that the drive shaft 22 and the projection 22a may be formed as respective members separate from each other.

The guide section 3 has an outer shell pipe 25, which forms an outer shell of the guide section 3, and the drive shaft 22 referred to above is positioned at a center of this outer shell pipe 25. The drive shaft 22 so positioned is rotatably supported by a plurality of rolling bearings 26 positioned spaced a distant apart from each other in a direction axially of the guide section 3. Between the neighboring rolling bearings 26, spring elements 27A for generating a preload on the inner rings of the corresponding rolling bearing 26 and spring elements 27B for generating the preload on the outer rings of the corresponding rolling bearings 26 are alternately disposed relative to each other. Those spring elements 27A and 27B may be employed in the form of, for example, compression springs. The constraint member 21 referred to previously is fixed to a pipe end portion 25a of the outer shell pipe 25 by means of a fixing pin 28 and has its distal end inner peripheral portion supporting a distal end of the drive shaft 22 through a rolling bearing 29. It is, however, to be noted that the pipe end portion 25a may be a member separate from the outer shell pipe 25 and may then be connected with the outer shell pipe 25 by means of, for example, welding.

Provided between an inner diametric surface of the outer shell pipe 25 and the drive shaft 22 is a guide pipe 30, having its opposite ends opening. Within a guide hole 30a which is an inner diametric hole of this guide pipe 30, an attitude altering or operating member 31 is reciprocally movably inserted. The wire for the attitude altering member is the same as that for the drive shaft 22 referred to previously. For a material for the wire, metal, resin or glass fiber, for example, can be employed. The wire may be either a single wire or a twisted wire. Also, a shape memory alloy may be employed for the wire. The attitude altering member 31 has a tip which is of a spherical shape, and, as shown in Fig. 2D, the spherical tip thereof is held in contact with a bottom surface of a radially extending groove portion 11b formed in the base end face of the housing 11. The groove portion 11b and the attitude altering member 31 altogether form a rotation preventing mechanism 37 for preventing the distal end member 2, shown in Fig. 2A, from rotating about the center line CL of the distal end member 2 relative to the guide section 3 when the tip portion of the attitude altering member 31, inserted in the groove portion 11b, is engaged with a side face of the groove portion 11b.

Between a base end face of the housing 11 of the distal end member 2 and a tip end face of the outer shell pipe 25 of the guide section 3, a counterforce applying unit 32 made of, for example, a compression coil spring, is arranged at a location spaced 180° degrees circumferentially in phase from the circumferential location where the attitude altering member 31 is positioned. This counterforce applying unit 32 is operable to apply a force, which counteracts against the force the selective advance or retraction of the attitude altering member 31 applies to the distal end member 2, to the distal end member 2.

Also, a plurality of reinforcement shafts 34 are arranged, separate from the guide pipe 30, between the inner diametric surface of the outer shell pipe 25 and the drive shaft 22 and on the same pitch circle C as that depicted by the guide pipe 30. Those reinforcement shafts 34 are employed for securing the rigidity of the guide section 3. The guide pipe 30 and the plural reinforcement shafts 34 are spaced an equal distance from each other. The guide pipe 30 and the plural reinforcement shafts 34 are held in contact with the inner diametric surface of the outer shell pipe 25 and an outer diametric surface of each of the rolling bearings 26 so as to support the respective outer diametric surfaces of the rolling bearings 26.

The outer shell pipe 25, the guide pipe 30 and the reinforcement shafts 34, all forming respective component parts of the guide section 3, are curved over their entire lengths and are so curved as to assume the same curved shape. The outer shell pipe 25, the guide pipe 30 and the reinforcement shafts 34, although they are of an arcuate shape in which various portions thereof have a uniform curvature in the instance as shown, may not be limited to the arcuate shape, but may have a shape represented by a quadratic curve or a curved shape similar to the quadratic curve.

For the above described attitude altering member 31, what has been plastically formed to have a curved shape following the curved shape of the guide hole 30a in the guide pipe 30 is employed. The attitude altering member 31 is made of a material of a kind capable of being elastically deformed to a certain extent, but is plastically deformed by a plastic forming process so as to have the above described curved shape under a natural condition where no external force acts. For the above described plastic deformation, a mold 50 for the plastic formation of the attitude altering member 31 is employed as shown in Fig. 4A. The mold 50 has a groove 51 of a sectional shape having a curvature greater than the curvature of the curved shape of the guide hole 30a and so selected as to enable the attitude altering member 31 to be engaged therein in a movement constrained fashion. For example, as shown in Fig. 4B, if the radius R1 of curvature of the guide hole 30a is chosen to be 300 mm, the radius R2 of curvature of the groove 51 is chosen to be 200 mm. By engaging the attitude altering member 31' as a straight material under the natural condition in the groove 51 of the mold 50 (Fig. 4A) retaining it for a predetermined length of time and plastically forming it, the attitude altering member 31 (Fig. 4B) is plastically deformed to a desired curved shape. The reason for the selection of the curvature of the groove 51, which is greater than the curvature of the curved shape of the guide hole 30a, is that since the attitude altering member 31, when removed out of the groove 51, will restore towards the original shape by a quantity it has been elastically deformed, the attitude altering member 31 need be curved too much by that quantity the attitude altering member 31 has been elastically deformed.

Fig. 3A illustrates the work took drive mechanism 4b and the attitude altering drive mechanism 4c both accommodated within the drive unit housing 4a. The work tool drive mechanism 4b includes the work tool drive source 41 adapted to be controlled by the controller 5. The work tool drive source 41 is in the form of, for example, an electrically driven motor having its output shaft 41a coupled with a base end of the drive shaft 22. The attitude altering drive mechanism 4c includes an attitude altering drive source 42 adapted to be controlled by the controller 5. This attitude altering dive source 42 is in the form of, for example, an electrically driven actuator, and the movement of an output rod 42a, which is a direct acting member capable of selectively advancing and retracting, i.e., reciprocally movable in a direction leftwards and rightwards as viewed in Fig. 3A, is transmitted to the attitude altering member 31 through a force increasing and transmitting mechanism 43.

The force increasing and transmitting mechanism 43 includes a pivot lever 43b pivotable about a support pin 43a and is so designed and so configured as to allow a force of the output rods 42a to work on a working point P1 of the levers 43b, which are respectively spaced a long distance from the support pin 43a, and as to apply a force to the attitude altering members 31 at a force point P2, which are spaced a short distance from the support pin 43a, wherefore the outputs of the attitude altering drive sources 42 can be increased and then transmitted to the attitude altering members 31. Since the use of the force increasing and transmitting mechanism 43 is effective to enable a large force to be applied to the attitude altering members 31 even in the linear actuator of a low output capability, the linear actuator can be downsized. The drive shaft 22 referred to above is made to extend through an opening 44 defined in the pivot lever 43b. It is also to be noted that in place of the use of, for example, the electrically driven actuator, the attitude of the distal end member 2 may be manually altered by remote control.

The attitude altering drive mechanism 4c is provided with an actuation amount detector 45 for detecting the amount of actuation of the attitude altering drive source 42. A detected value of this actuation amount detector 45 is fed to an attitude detector 46. The attitude detector 46 then detects an inclined attitude of the distal end member 2 about the X axis (Fig. 2B) in reference to an output of the actuation amount detector 45. The attitude detector 46 includes a relation setting unit (not shown), in which relations between the inclined attitude and the input signal of the actuation amount detector 45 are set in terms of calculating equations and/or tables so that the inclined attitude can be detected in reference to the inputted output signal with the use of the relation setting unit. It is to be noted that this attitude detector 46 may be provided either inside the controller 5 or in an external control device.

The attitude altering drive mechanism 4c is provided with a supply power meter 47 for detecting the electric energy supplied to the attitude altering drive source 42, which is an electrically operated actuator, independent of each other. The detection value of this supply power meter 47 is outputted to a load detector 48. This load detector 48 in turn detects a load acting on the distal end member 2 in reference to the outputs of the supply power meter 47. Specifically, this load detector 48 includes a relation setting unit (not shown), in which the relation between the load and the output signal of the supply power meter 47 is set in terms of an arithmetic equation or table, and makes use of the relation setting unit to detect the load in reference to the output signal so inputted. This load detector 48 may be provided either in the controller 5 or in an external control device.

The controller 5 is provided with a tool rotation operating instrument 5a for outputting a rotation command signal of the tool 1 and an attitude alteration operating instrument 5b for outputting an attitude alteration command signal of the distal end member 2. This controller 5 has an electronic calculating circuit (not shown) and a control program (not shown) built therein and is operable to control the work tool drive source 41 and the attitude altering drive source 42 on the basis of the respective command signals from the tool rotation operating instrument 5a and the attitude alteration operating instrument 5b and respective output signals from the attitude detector 46 and the load detector 48.

The operation of the remote controlled actuator of the structure described hereinabove will now be described. When by operating the tool rotation operating instrument 5a the work tool drive source 41 as shown in Fig. 3A is driven, the rotational force thereof is transmitted to the spindle 13 through the drive shaft 22 to thereby rotate the tool 1 together with the spindle 13. The load acting on the distal end member 2 when the tool 1 then being rotated cuts a bone or the like is estimated from the detection value of the supply power meter 47, shown in Fig. 3A, by the load detector 48. Accordingly, when the amount of feed of the remote controlled actuator in its entirety and the alteration of attitude of the distal end member 2, as will be described later, are controlled in dependence on the value of the load detected in the manner described above, cutting of the bone can be properly carried out while the load acting on the distal end member 2 is maintained properly.

Also, when the attitude alteration operating instrument 5b is operated to drive the attitude altering drive source 42, the attitude altering member 31 is selectively advanced or retracted to alter the attitude of the distal end member 2. By way of example, if the attitude altering member 31 is advanced by the attitude altering drive source 42 in a direction towards the tip or distal side, the housing 11 for the distal end member 2 is pressed by the attitude altering member 31 with the distal end member 2 consequently altered in attitude along the guide faces F1 and F2 so that the tip or distal side can be oriented downwardly as viewed in Fig. 2A. If the attitude altering member 31 is conversely retracted by the attitude altering drive source 42, the housing 11 for the distal end member 2 is pressed backwardly by the effect of the elastic repulsive force exerted by the counterforce applying unit 32 and, consequently, the distal end member 2 is altered in attitude along the guide faces F1 and F2 so that the tip or distal side can be oriented upwardly as viewed in Fig. 2A. At this time, a pressure from the attitude altering member 31, the elastic repulsive force from the counterforce applying unit 32 and a reactive force from the constraint member 21 are applied to the distal end member coupling unit 15 and, depending on the balance of those applied forces, the attitude of the distal end member 2 is determined. The attitude of the distal end member 2 is detected by the attitude detector 46 from the detection value of the actuation amount detector 45. For this reason, the attitude of the distal end member 2 can be properly controlled by remote control.

Since the attitude altering member 31 is inserted through the guide hole 30a of the guide pipe 30, the attitude altering member 31 can properly act on the distal end member 2 at all times without being accompanied by displacement in position in a direction perpendicular to the lengthwise direction thereof and the attitude altering operation of the distal end member 2 can therefore be performed accurately. Also, since the center of the junction between the spindle 13 and the drive shaft 22 lies at the same position as the respective centers of curvature O of the guide faces F1 and F2, no force tending to press and pull will not act on the drive shaft 22 as a result of the alteration of the attitude of the distal end member 2 and the distal end member 2 can be smoothly altered in attitude.

If, as shown in Fig. 5A, the attitude altering member 31 of a wire-like shape, which is straight under a natural condition, is inserted into the guide hole 30a of a curved shape for selective advance and retraction, the frictional forces F_{A2}, F_{B2} and F_{C2} occur between the attitude altering member 31 and the inner wall surface of the guide hole 30a, as hereinbefore described, during the advance or retraction of the attitude altering member 31. However, in the embodiment hereinabove described, since the attitude altering member 31 is, when has not yet been inserted into the guide hole 30a and is under the natural condition, of a curved shape similar to the curved shape of the guide hole 30a, as shown in Fig. 5B, the frictional force between the attitude altering member 31 and the inner wall surface of the guide hole 30a, when in a condition with the attitude altering member 31 having been inserted into the guide hole 30a, is so low that extension or contraction of the attitude altering member 31 will hardly occur. For this reason, an error brought about by the expansion or contraction of the attitude altering member 31 is small and a highly accurate control to alter the attitude of the distal end member 2 can be accomplished. Also, since the frictional forces between the attitude altering member 31 and the inner wall surface of the guide hole 30a are low, no large force is required to selectively advance or retract the attitude altering member 31 and, therefore, the use of a small sized attitude altering drive source 42 is sufficient.

In addition, since the use has been made of the rotation preventing mechanism 37 for preventing the distal end member 2 from rotating about the center line CL of the distal end member 2 relative to the guide section 3, even in the event that the distal end member 2 for holding the tool 1 becomes uncontrollable because of, for example, a failure of one or both of the attitude altering drive mechanism 4c for controlling the selective advance and retraction of the attitude altering member 31 and a control device therefor, the risk can be avoided that the distal end member 2 will rotate about the center line CL to impair the surroundings of the site to be processed and/or the distal end member 2 itself may be damaged.

The remote controlled actuator of the foregoing construction is utilized in grinding the femoral marrow cavity during, for example, the artificial joint replacement surgery and during the surgery, it is used with the distal end member 2 in its entirety or a part thereof inserted into the body of a patient. Because of this, with such distal end member 2 as described above that can be altered in attitude by remote control, the bone can be processed in a condition with the tool 1 maintained in a proper attitude at all times and the opening for insertion of the artificial joint can be finished accurately and precisely.

There is the necessity that the drive shaft 22 and the attitude altering member 31 are provided within the guide section 3 of an elongated shape in a protected fashion. Hence, the drive shaft 22 is provided in the center portion of the outer shell pipe 25 and the guide pipe 30, in which the attitude altering member 31 is accommodated, and the reinforcement shafts 34 are arranged between the outer shell pipe 25 and the drive shaft 22 so as to be juxtaposed in the circumferential direction. Accordingly, it is possible to protect the drive shaft 22 and the attitude altering member 31 and, at the same time, the interior can be made hollow to thereby reduce the weight without sacrificing the rigidity. Also, the balance as a whole is good.

Since the outer diametric surfaces of the rolling bearings 26 supporting the drive shaft 22 are supported by the guide pipes 30 and the reinforcement shafts 34, the outer diametric surfaces of the rolling bearings 26 can be supported with no need to use any extra member. Also, since the preload is applied to the rolling bearings 26 by means of the spring elements 27A and 27B, the drive shaft 22 comprised of the wire can be rotated at a high speed. Because of that, the processing can be accomplished with the spindle 13 rotated at a high speed and a good finish of the processing can also be obtained and the cutting resistance acting on the tool 1 can be reduced. Since the spring elements 27A and 27B are disposed between the neighboring rolling bearings 26, the spring elements 27A and 27B can be provided with no need to increase the diameter of the guide section 3.

In the embodiment described hereinbefore, the work tool drive source 41 and the attitude altering drive source 42 are provided within the common drive unit housing 4a. For this reason, the structure of the remote controlled actuator as a whole can be simplified. It is, however, to be noted that only one of the work tool drive source 41 and the attitude altering drive source 42 may be provided within the drive unit housing 4a. Also, as will be explained later, both of the work tool drive source 41 and the attitude altering drive source 42 may be provided outside the drive unit housing 4a.

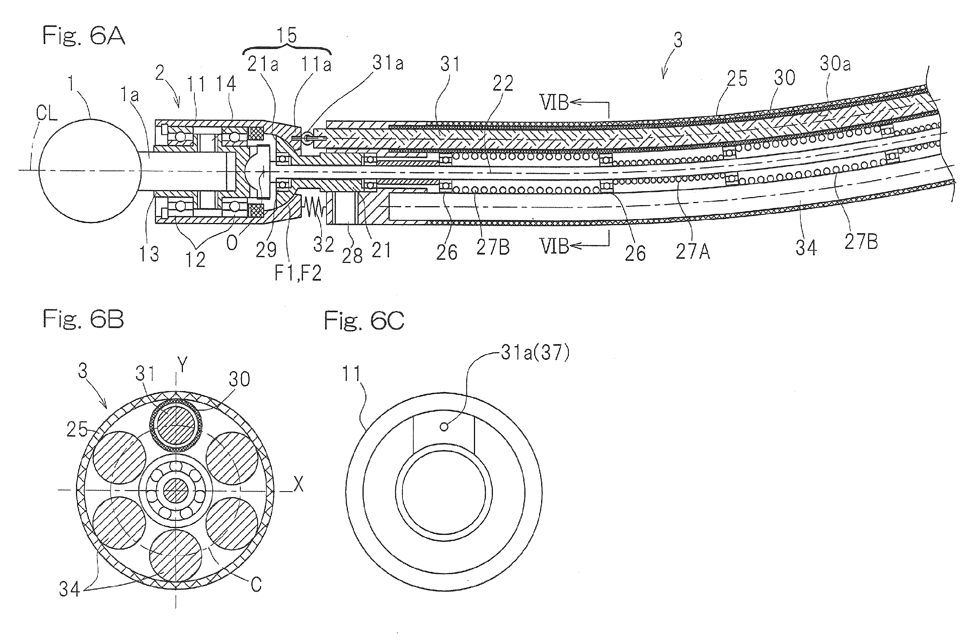

Although in the first embodiment as hereinbefore described, the alteration of the attitude of the distal end member 2 has been shown and described as accomplished by causing the attitude altering member 31 to press the housing 11, arrangement may be made that as is the case with a second preferred embodiment of the present invention shown in Figs. 6A to 6C, the tip of the wire-like attitude altering member 31 and the housing 11 are connected together through a connecting member 31a so that when the attitude altering member 31 is retracted by an attitude altering drive source (not shown) towards the tip side, the attitude altering member 31 can pull the housing 11 to cause the distal end member 2 to alter the attitude. In such case, a coiled tension spring is used for the counterforce applying unit 32.

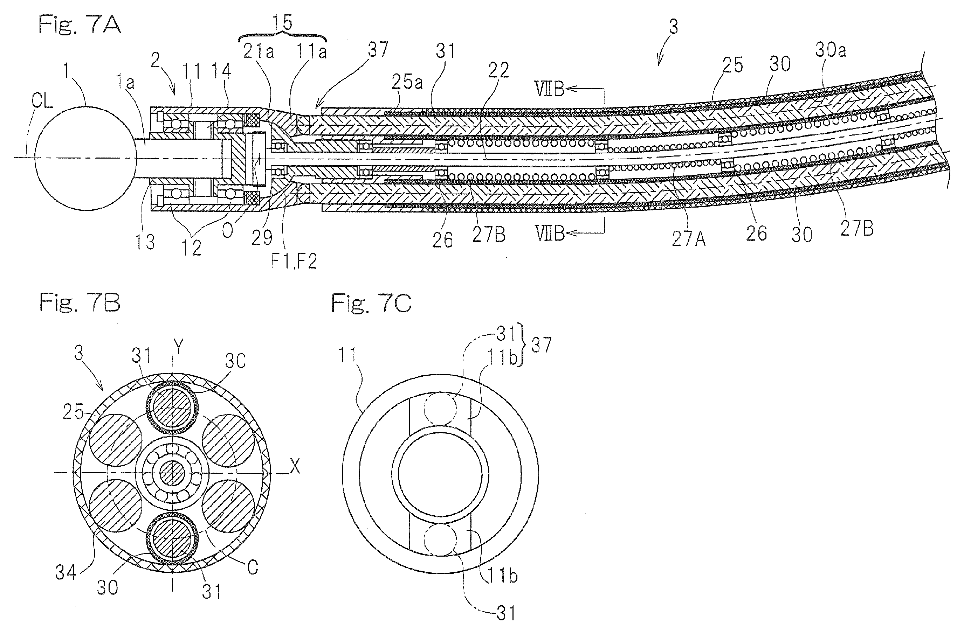

Figs. 7A to 7C illustrate a third preferred embodiment of the present invention. The remote controlled actuator according to this embodiment is of such a structure that, as best shown in Fig. 7B, two guide pipes 30 are provided within the outer shell pipe 25 at respective circumferential locations spaced 180° in phase from each other in a direction circumferentially of the outer shell pipe 25, and the attitude altering member 31 is inserted in a guide hole 30a of each of the guide pipes 30, which is an inner diametric hole of the corresponding guide pipe 30, for selective advance or retraction. Between those two guide pipes 30, a plurality of reinforcement shafts 34 are arranged on the same pitch circle C as that of the guide pipes 30. The guide faces F1 and F2 best shown in Fig. 7A are represented by spherical surfaces having a center of curvature at a point O or cylindrical faces having its longitudinal axis represented by the X-axis passing through the point O. Also, as best shown in Fig. 7C, the spherical tip of each of the attitude altering members 31 is held in contact with a bottom face of a radially extending groove 11b, defined in the base end face of the housing 11, and the groove 11b and the attitude altering member 31 cooperate with each other to define the rotation preventing mechanism 37 operable to prevent the distal end member 2 from rotating about the center line CL of the distal end member 2 relative to the guide section 3.

The drive unit 4 (not shown) is provided with two attitude altering drive sources 42 (not shown) for selectively advancing and retracting respective attitude altering members 31 so that when those two attitude altering drive sources 42 are driven in respective directions opposite to each other, the distal end member 2 can be altered in attitude. By way of example, when the upper attitude altering member 31 shown in Fig. 7A is advanced towards the tip end side and the lower attitude altering member 31 is retracted, the housing 11 for the distal end member 2 is pressed by the upper attitude altering member 31 and, therefore, the distal end member 2 is altered in attitude along the guide surfaces F1 and F2 with the tip end side oriented downwards as viewed in Fig. 7A. Conversely, when both of the attitude altering members 31 are driven in the directions opposite thereto, the lower attitude altering member 31 urges the housing 11 for the distal end member 2 to allow the distal end member 2 to alter in attitude along the guide surfaces F1 and F2 with the distal end side oriented upwardly as viewed in Fig. 7A.

As hereinabove described, even when the two attitude altering members 31 arranged spaced 180° in phase from each other are advanced and retracted in respective directions opposite to each other, the attitude of the distal end member 2 can be altered. During this alteration, when one of the two attitude altering members 31 applies a force necessary to alter the attitude, the other of the two attitude altering members 31 functions as the counterforce applying unit for applying a force, which counteracts against the force necessary to alter the attitude, to the distal end member.

The pressures from the upper and lower attitude altering members 31 and a reactive force from the constraint member 21 act on the distal end member connecting unit 15 and, accordingly, the attitude of the distal end member 2 is determined in dependence on the balance of those working forces. According to this construction, since the housing 11 for the distal end member 2 is pressed by the two attitude altering members 31, as compared with the previously described embodiment in which it is pressed by a single attitude altering member 31, the attitude stability of the distal end member 2 can be increased.

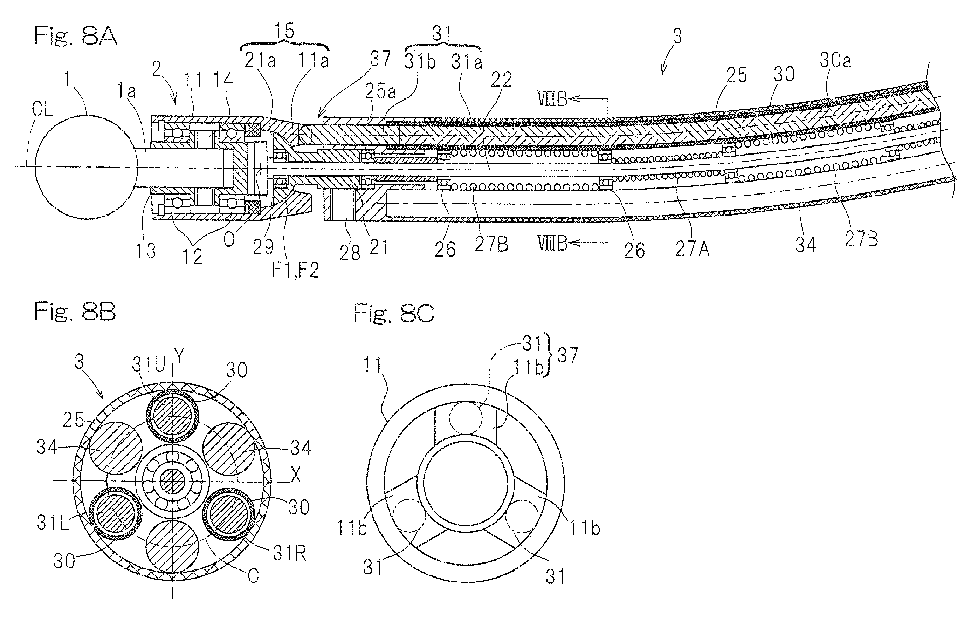

A fourth preferred embodiment of the present invention is shown in Figs. 8A to 8C. In this remote controlled actuator, as best shown in Fig. 8B, three guide pipes 30 are employed and arranged at respective circumferential locations spaced 120° in phase from each other within the outer shell pipe 25, and the attitude altering member 31 is reciprocally movably inserted in each of the guide holes 30a, which are inner diametric holes of such guide holes 30a, in a manner similar to that described hereinbefore. Among the three guide pipes 30, a plurality of reinforcement shafts 34 are arranged on the same pitch circle C as that of the guide pipes 30. No resilient restoring member 32 is employed. As shown in Fig. 8A, the guide faces F1 and F2 represent spherical faces having a center of curvature at a point O and the distal end member 2 can be tilted in any arbitrarily chosen direction. In such case, as best shown in Fig. 8C, the groove 11b of the housing 11 and the attitude altering members 31 cooperate with each other to define the rotation preventing mechanism 37 for preventing the distal end member 2 from rotating about the center line CL relative to the guide section 3.

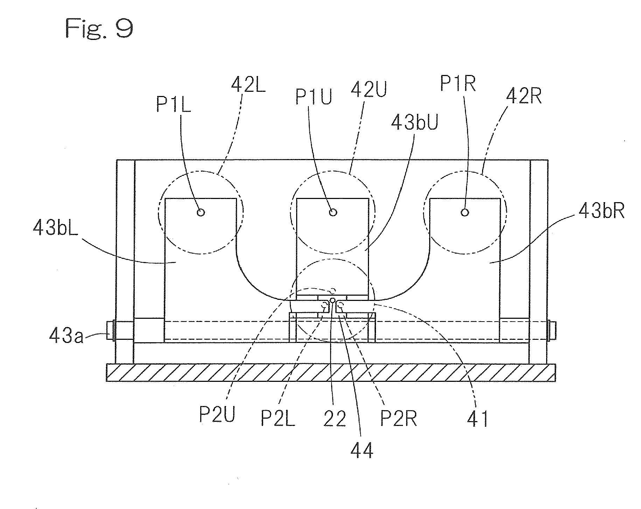

The drive unit 4 is provided with three attitude altering drive sources 42 (42U, 42L and 42R), best shown in Fig. 9, for selectively advancing or retracting respective attitude altering members 31 (31U, 31L and 31R) and, accordingly, the attitude of the distal end member 2 is altered by driving those three attitude altering drive sources 42 in liaison with each other.

By way of example, when one of the attitude altering members 31U, upper side one as viewed in Fig. 8A, is advanced towards the distal, tip end side while the other two attitude altering members 31L and 31R are retracted, the housing 11 for the distal end member 2 is pressed by the upper attitude altering member 31U to allow the distal end member 2 to be altered in attitude along the guide surfaces F1 and F2 with the distal, tip end side consequently oriented downwardly as viewed in Fig. 8A. At this time, those attitude altering drive sources 42 are controlled so that the amount of advance or retraction of each of the attitude altering members 31 may become proper. On the other hand, when each of those attitude altering members 31 is retracted or advanced, the housing 11 for the distal end member 2 is pressed by the attitude altering members 31L and 31R, which are shown on lower left and lower right sides, and, consequently, the distal end member 2 is altered in attitude along the guide surfaces F1 and F2 with the tip end side oriented upwardly as viewed in Fig. 8A.

Also, when while the attitude altering member 31U on the upper side is held still, the attitude altering member 31L on the left side is advanced towards the tip end side and the attitude altering member 31R on the right side is retracted, the housing 11 for the distal end member 2 is pressed by the attitude altering member 31L on the left side to allow the distal end member 2 to be oriented rightwards, that is, to be altered in attitude along the guide surfaces F1 and F2 with the distal end member 2 oriented towards a rear side of the sheet of the drawing of Fig. 8A. Conversely, when the attitude altering members 31L and 31R on the left and right sides are advanced and retracted, the housing 11 for the distal end member 2 is pressed by the attitude altering member 31R on the right side, allowing the distal end member 2 to be altered in attitude so that the distal end member 2 can be guided along the guide surfaces F1 and F2 so as to be oriented leftwards.

When the attitude altering members 31 are arranged at the three locations in the circumferential direction as described above, it is possible to alter the attitude of the distal end member 2 in two-axis directions (X axis and Y axis), that is, vertical and horizontal directions. At the time of this attitude alteration, when the force necessary to alter the attitude of the distal end member 2 is applied to the distal end member 2 by one or two of the three attitude altering members 31, the remaining two or one of the three attitude altering members 31 function as the counterforce applying unit for applying the force, which counteracts against the force necessary to alter the attitude, to the distal end member.

Respective pressures from the three attitude altering members 31 and the reactive force from the constraint member 21 act on the distal end member connecting unit 15 and, therefore, the attitude of the distal end member 2 is determined in dependence on the balance of those working forces. According to the above described construction, since the housing 11 for the distal end member 2 is pressed by the three attitude altering members 31, the attitude stability of the distal end member 2 can be further increased. If the number of the attitude altering members 31 is increased, the attitude stability of the distal end member 2 can be yet further increased.

Where the attitude altering members 31 are provided at the three circumferential locations such as shown in Fig. 8B, the attitude altering drive mechanism 4c may be so constructed as shown in, for example, Fig. 9. In other words, the three attitude altering drive sources 42 (42U, 42L and 42R) for selectively advancing or retracting the respective attitude altering members 31 (31U, 31L and 31R) are arranged leftwards and rightwards in parallel to each other and, at the same time, the lever 43b (43bU, 43bL and 43bR) corresponding to each of the attitude altering drive sources 42 is pivotally mounted around a common support pin 43a. Further, The force increasing and transmitting mechanism 43 is so designed and so configured as to allow a force of the output rods 42a (42aU, 42aL, 42aR) of the respective attitude altering drive sources 42 to work on a working point P1 (P1U, P1L, P1R) of the levers 43b, which are respectively spaced a long distance from the support pin 43a, and as to apply a force to the attitude altering members 31 at a force point P2 (P2U, P2L, P2R), which are spaced a short distance from the support pin 43a. Accordingly, the outputs of the attitude altering drive sources 42 can be increased and then transmitted to the attitude altering members 31. Since the use of the force increasing and transmitting mechanism 43 is effective to enable a large force to be applied to the attitude altering members 31 even in the linear actuator of a low output capability, the linear actuator can be downsized. It is to be noted that the drive shaft 22 is extended through an opening 44 defined in the pivot lever 43bU associated drivingly with the upper attitude altering member 31U.

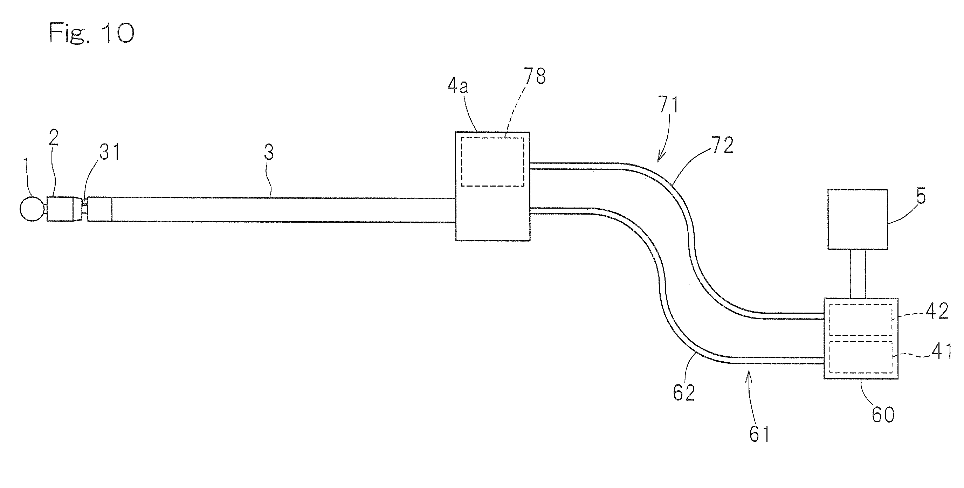

Figs. 10 to 13 illustrate a fifth preferred embodiment of the present invention, in which the work tool drive mechanism and the attitude altering drive mechanism are different from those of the first to forth embodiments described previously. Whereas in any one of the previously described embodiments, the work tool drive source 41 of the work tool drive mechanism 4b and the attitude altering drive source 42 of the attitude altering drive mechanism 4c have been shown and described as provided within the drive unit housing 4a, in the fifth embodiment shown in Figs. 10 to 13 the work tool drive source 41 and the attitude altering drive source 42 are provided in a drive unit housing 60 which is a member separate from the drive unit housing 4a.

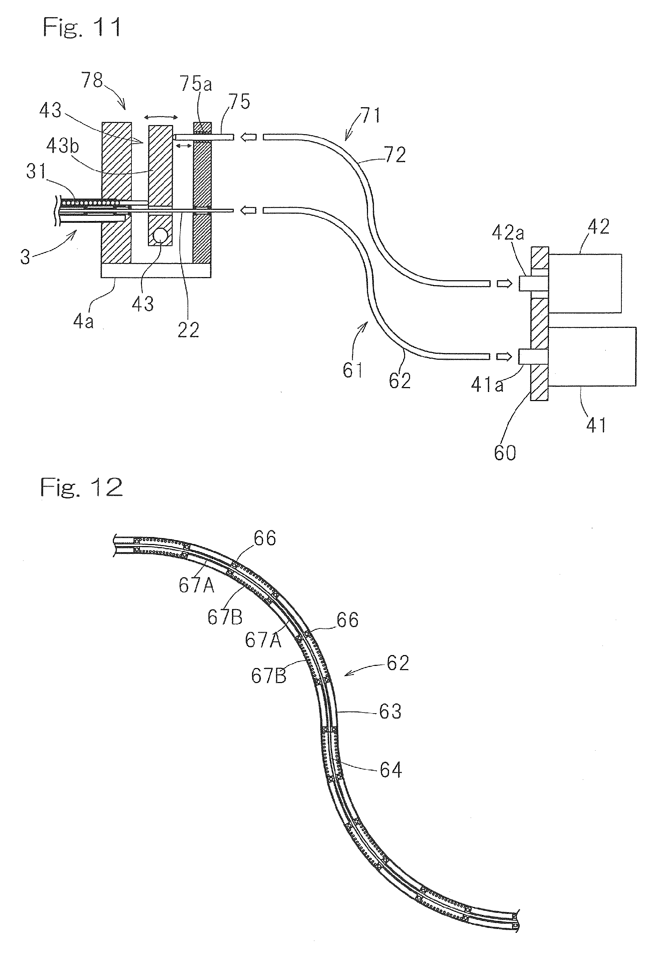

The tool rotation drive mechanism, now identified by 61, that is employed in the practice of this fifth embodiment is operable to transmit the rotation of the output shaft 41a of the tool rotation drive source 41, provided in the drive source housing 60, to a base end of the drive shaft 22 within the drive unit housing 4a by means of an inner wire 64 (shown in Fig. 12) of a work tool rotating cable 62. For example, the work tool rotating cable 62 may have such a structure as shown in Fig. 12. In other words, at a center of a flexible outer tube 63, the flexible inner wire 64 referred to above is rotatably supported by a plurality of rolling bearings 66. The inner wire 64 has its opposite ends connected respectively with base ends of the output shaft 41a of the tool rotation drive source 41 and the drive shaft 22. Between the neighboring rolling bearings 66, spring elements 67A and 67B are provided for generating preloads to the rolling bearings 66. Those spring elements 67A and 67B are employed in the form of, for example, compression coil springs. Those spring elements include an inner ring spring element 67A for applying the preload to an inner ring of each of the rolling bearings 66 and an outer ring spring element 67B for applying the preload to an outer ring of each of the rolling bearings 66, and those spring elements 67A and 67B are disposed alternately relative to each other. With the preloads having been applied from the spring elements 67A and 67B to the rolling bearings 66 in this way, the inner wire 64 can be rotated at a high speed. A commercially available flexible shaft may be employed for the work tool rotating cable 62.

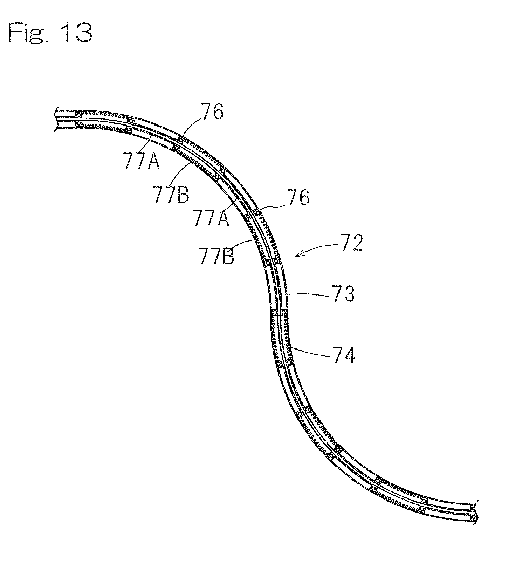

Also, the attitude altering drive mechanism 71 employed in the practice of the fifth embodiment is of such a structure that the operation of the attitude altering drive source 42 provided in the drive source housing 60 is transmitted to a drive mechanism section 78 within the drive unit housing 4a through an attitude alteration cable 72. The drive mechanism section 78 corresponds to the attitude altering drive mechanism 4c, which is employed in the practice of the previously described embodiment and from which the attitude altering drive source 42 is removed, and, in place of the output rod 42a of the attitude altering drive source 42 in the attitude altering drive mechanism 4c, the use is made of an advancing and retracting member 75 capable of selectively advancing or retracting relative to the drive unit housing 4a in a condition with its tip held in contact with the pivot lever 43b. The advancing and retracting member 75 is, when the rotary motion is translated into a linear motion by a screw mechanism 75a such as, for example, a ball screw, selectively advanced or retracted relative to the drive unit housing 4a. In such case, the attitude altering drive source 42 is in the form of a rotary actuator and the rotation of this attitude altering drive source 42 is transmitted to the advancing and retracting member 75 by means of the inner wire 74 (Fig. 13) of the attitude alteration cable 72.

The attitude altering cable 72 is of the same structure as the work tool rotating cable 62 and takes such a structure as shown in Fig. 13. In other words, at a center of a flexible outer tube 73, the flexible inner wire 74 is rotatably supported by a plurality of rolling bearings 76. The inner wire 74 has its opposite ends connected respectively with the output shaft 42a of the attitude altering drive source 42 and a selective advancing and retracting member 75. Between the neighboring rolling bearings 76, spring elements 77A and 77B are provided for generating preloads to the rolling bearings 76. Those spring elements 77A and 77B are employed in the form of, for example, compression coil springs. There are an inner ring spring element 77A for applying the preload to an inner ring of each of the rolling bearings 76 and an outer ring spring element 77B for applying the preload to an outer ring of each of the rolling bearings 76, and those spring elements 77A and 77B are disposed alternately relative to each other. With the preloads having been applied from the spring elements 77A and 77B to the rolling bearings 76 in this way, the inner wire 74 can be rotated at a high speed. A commercially available flexible shaft may be employed for the attitude altering cable 72.

As shown in Fig. 10, the controller 5 for controlling the work tool drive source 41 and the attitude altering drive source 42 is connected with the drive source housing 60. Each of the distal end member 2 and the guide section 3 is of a structure identical with that employed in the practice of any one of the previously described embodiments.

If, as is the case with the foregoing fifth embodiment, the work tool drive source 41 and the attitude altering drive source 42 are provided outside the drive unit housing 4a, the drive unit housing 4a can be compactized. For this reason, the handleability at the time the remote controlled actuator is operated with the drive unit housing 4a carried by an operator can be increased.

Although the present invention has been fully described in connection with the preferred embodiments thereof with reference to the accompanying drawings which are used only for the purpose of illustration, those skilled in the art will readily conceive numerous changes and modifications within the framework of obviousness upon the reading of the specification herein presented of the present invention. By way of example, although in any one of the embodiments of the present invention hereinbefore fully described, the work tool has been shown and described as represented by a tool 1, the work tool may be any other work tool such as, for example, a prehension orthosis.

Also, the present invention may not be necessarily limited to the remote controlled actuator for medical use, but can be equally applied to any other remote controlled actuator that is used in any other field such as, for example, a mechanical processing field.

Accordingly, such changes and modifications are, unless they depart from the scope of the present invention as delivered from the claims annexed hereto, to be construed as included therein.

### [Reference Numerals]

- 1 ····: Tool (Work Tool)
- 2 ····: Distal end member
- 3 ····: Guide section
- 4a ····: Drive unit housing
- 15 ····: Distal end member connecting unit
- 22 ····: Drive shaft
- 26, 29 ····: Rolling bearing
- 27A, 27B ····: Spring element
- 30 ····: Guide pipe
- 30a ····: Guide hole
- 31 ····: Attitude altering member
- 32 ····: Counterforce applying unit
- 41 ····: Work tool drive source
- 42 ····: Attitude altering drive source
- 50 ····: Mold
- 51 ····: Groove

## Claims

1. A remote controlled actuator which comprises:
an elongated guide section of a curved shape;
a distal end member fitted to a tip of the guide section for alteration in attitude;
a work tool provided in the distal end member;
a work tool drive source for driving the work tool; and
an attitude altering drive source for operating the attitude of the distal end member,
in which the guide section has an interior, in which a drive shaft for transmitting a driving force of the work tool drive source to the work tool and a curved guide hole extending to its opposite ends are accommodated, and a wire-like attitude altering member is inserted within the guide hole for selective advance or retraction, the wire-like attitude altering member being adapted to be driven by the attitude altering drive source to undergo a selective advance or retraction to alter the attitude of the distal end member; and
in which the attitude altering member has a curved shape similar to the curved shape of the guide hole under a natural condition while the attitude altering member has not yet been inserted into the guide hole.

2. The remote controlled actuator as claimed in claim 1, in which the attitude altering drive source is operable to selectively advance or retract the attitude altering member so that the tip of the attitude altering member may press the distal end member,
further comprising a counterforce applying unit for applying a force to the distal end member, which force counteracts against a force applied to the distal end member as a result of the selective advance or retraction of the attitude altering member,
wherein the attitude of the distal end member is altered and maintained by the balance between the force, which the attitude altering member applies to the distal end member, and the force which the counterforce applying unit applies to the distal end member.

3. The remote controlled actuator as claimed in claim 1, in which the attitude altering drive source is operable to selectively advance or retract the attitude altering member so that the tip of the attitude altering member may pull the distal end member,
further comprising a counterforce applying unit for applying a force to the distal end member, which force counteracts against a force applied to the distal end member as a result of the selective advance or retraction of the attitude altering member,
wherein the attitude of the distal end member is altered and maintained by the balance between the force, which the attitude altering member applies to the distal end member, and the force which the counterforce applying unit applies to the distal end member.

4. The remote controlled actuator as claimed in claim 1, in which the attitude altering member is shaped to the same shape as the curved shape of the guide hole by means of a plastic deformation caused by a plastic forming.

5. The remote controlled actuator as claimed in claim 1, in which the attitude altering member is shaped to the same shape as the curved shape of the guide hole by means of a cutting process.

6. The remote controlled actuator as claimed in claim 1, in which the drive shaft is a rotary shaft,
further comprising a plurality of rolling bearings for rotatably supporting the drive shaft within the guide section; and a spring element provided between the neighboring rolling bearings for applying a preload to those rolling bearings.

7. The remote controlled actuator as claimed in claim 1, in which the distal end member is connected with the tip of the guide section through a distal end member connecting unit for tilting in an arbitrary direction; each of the guide hole and the attitude altering member, inserted into the guide hole, is provided at three or more locations about a center of tilting of the distal end member; the attitude altering drive source is provided for each of the attitude altering members; and the attitude of the distal end member is altered and maintained in two axis directions by the balance between respective forces which the attitude altering members at those three or more locations apply to the distal end member.

8. The remote controlled actuator as claimed in claim 1, in which one or both of the work tool drive source and the attitude altering drive source is/are provided within a drive unit housing to which a base end of the guide section is connected.

9. The remote controlled actuator as claimed in claim 1, in which the work tool drive source and the attitude altering drive source are provided outside the drive unit housing to which a base end of the guide section is connected.

10. A plastic forming method for making the attitude altering member for use in the remote controlled actuator as described in claim 4, in which using a mold having a groove of a sectional shape with a curvature greater than the curvature of the curved shape of the guide hole and capable of accommodating the attitude altering member in a non-movable fashion, the attitude altering member, which is straight under a natural condition, being plastically deformed while accommodated within the groove in the mold.
